Europäisches Patentamt

(19) European Patent Office

Office européen des brevets

(11) EP 0 654 984 B1

(12) **EUROPEAN PATENT SPECIFICATION**

(45) Date of publication and mention
of the grant of the patent:
**04.11.1998 Bulletin 1998/45**

(21) Application number: **92918619.5**

(22) Date of filing: **14.08.1992**

(51) Int. Cl.$^6$: **A61F 9/00**, B23K 26/06

(86) International application number:
**PCT/US92/06864**

(87) International publication number:
**WO 94/04107 (03.03.1994 Gazette 1994/06)**

(54) **ASTIGMATIC LASER ABLATION OF SURFACES**

ASTIGMASTISCHE LASEROBERFLÄCHENABLATION

ABLATION ASTIGMATE DE SURFACES PAR LASER

(84) Designated Contracting States:
**DE ES FR GB IE IT NL**

(43) Date of publication of application:
**31.05.1995 Bulletin 1995/22**

(73) Proprietor:
**SUMMIT TECHNOLOGY, INC.**
**Waltham, Massachusetts 02154 (US)**

(72) Inventors:
- **SEILER, Theo**
**University Eye Clinic**
**P.O. Box 388 D-1000 Berlin 19 (DE)**

- **KLOPOTEK, Peter, J.**
**Framingham, MA 01701 (US)**

(74) Representative:
**Greenwood, John David et al**
**Graham Watt & Co.**
**Riverhead**
**Sevenoaks Kent TN13 2BN (GB)**

(56) References cited:
**EP-A- 0 296 982      EP-A- 0 429 368**
**EP-A- 0 529 822      US-A- 4 941 093**

- **PATENT ABSTRACTS OF JAPAN vol. 14, no. 159 (M-956)1990 & JP,A,2 020 681 ( FUJITSU ) 24 January 1990**

## Description

### Background of the Invention

This invention relates generally to methods and apparatus for eroding or ablating surfaces by lasers. In particular, this invention relates to methods and apparatus for eroding surfaces astigmatically and for the correction of myopic astigmatism through laser keratoplasty or keratomileusis.

Lasers can etch surfaces in a controlled manner. These techniques are known and depend upon the interaction between the surface structure and the laser radiation which has a known wavelength and energy density. In addition, the ablation of a surface by laser radiation is typically time-rate dependent, although this time dependency is usually non-linear with ablative depth because of heat build-up and other artifacts. Therefore, ablative precision is often obtained through the use of pulsed laser radiation. Short pulses provide controlled depth etching in the application area. This control is especially important in keratoplasty or in keratomileusis procedures where the cornea of the eye is ablated to correct certain visual deficiencies, such as near-sightedness. By careful application of laser radiation to the cornea, physicians are able to surgically enhance a patient's vision with precision and without tissue damage by overexposure.

By successive application of laser pulses to a surface and by altering the size of the area under irradiation, curvatures can be created or altered on a surface. For example, when the ablative technique begins by irradiating a large, circular area and progressively decreases the radius of the exposed area, the central region has the greatest ablative depth because it is irradiated the longest. Conversely, the outer-most regions have the least ablative depth. If the surface is initially flat, the resulting profile would be concave. If the surface is initially convex, the surface can be made flat, or the curvature can be reduced depending upon the degree of ablation. In keratoplasty or in keratomileusis, the curvature on the cornea, i.e., the difference between the cornea's ideal curvature and the patient's actual corneal shape, is modified.

Surface erosion techniques to create or remove astigmatic shapes has been less successful. An astigmatic surface is by definition bi-powered: there are two natural and orthogonal curvatures creating the surface. A varying, circular irradiation pattern can only create or remove a single power on a surface. Existing methods to erode astigmatic shapes have been complex and difficult to implement.

For example, U.S. Patent No. 4,665,913 entitled "Method For Ophthalmological Surgery" discloses methods which provide for different astigmatic shapes through the use of laser scanning. However, this approach is especially difficult to control. The precise control of a reprofiling operation on a surface, e.g., a cornea, using a scanning laser, requires that the laser spot maintain nearly constant spatial intensity and further that the complex etching pattern is carefully followed. However, laser spot intensities are mostly Gaussian and inherently non-uniform. This non-uniformity and the detailed etching pattern leave much room for error, and, thus, require expensive safeguards for surgical applications. Additionally, these methods are naturally time-consuming, since only a small portion of the surface is ablated at a given time.

Another approach involves the use of a graded intensity or photodecomposable mask which varies the laser transmission to the target surface, thereby inducing variable ablative depths on the surface. For example, U.S. Patent No. 4,856,513 entitled "Laser Reprofiling Systems And Methods" which describes methodology for selectively eroding the cornea through the use of an erodable mask. The mask absorbs the surface laser radiation in varying amounts across the corneal surface to provide the desired surface profiles. This technique though requires the manufacture of a complementary object, i.e., the erodable mask, and also requires precise correlative positioning over the target surface.

Another apparatus and method for eroding an astigmatic surface is provided in U.S. Patent No. 4,941,093 entitled "Surface Erosion Using Lasers." According to its teachings, specifically configured optical elements or slits are used to provide ablation in one axis, i.e., such that the erosion proceeds selectively relative to a line rather than around a point. Typically, this approach requires a second step to provide spherical correction so that the proper overall curvature is achieved.

The apparatus of Patent Abstracts of Japan, Vol. 14, No. 159 (M-956), March 28, 1990 is as recited in the preamble of claim 3. The apparatus of the present invention is characterized by the features of the characterizing portion claim 3.

The method of the present invention is defined in claim 1.

It is, accordingly, an object of this invention to provide a simpler method and apparatus for astigmatically reprofiling a surface with an initial, bi-powered, astigmatic shape in order to achieve a new, preferably spherical, shape.

It is another object of this invention to provide a method and apparatus for orienting and adjusting the astigmatic ratio applied to a surface shape with increased control.

It is further an object of this invention to provide a method and apparatus for correcting myopic astigmatism through corneal ablation in laser keratomileusis procedures.

These and other objects of the invention are evident in the description that follows.

## Summary of the Invention

Apparatus and methods are disclosed for astigmatically ablating surfaces in order to impart new profiles and curvatures to such surfaces. A laser means, e.g., a rapidly pulsed laser radiation source, is aligned with a surface to provide photoablative pulses of energy along an optical path to a target region on the surface. A light restricting means, such as an adjustable iris or profiled mask, is disposed within the optical path to spatially control the extent of the laser radiation striking the target surface. Within the optical path, a cylindrical, optical system is also disposed and adjusted to modify the symmetrical laser beam into an elliptical shape for delivery to the target surface.

According to another aspect of the invention, the cylindrically-powered, optical system is rotatable about the laser beam axis. A rotation means is provided for orienting the elliptical shape to a selected axis on the target surface.

According to the invention, the cylindrically-powered, optical system is movable along the axis of the laser beam to modify one axis of the ellipse applied to the target surface to provide for differing astigmatic ratios.

Another aspect of the invention provides the further step of selecting the focal length of the cylindrically-powered, optical system, thereby allowing for the selection of varying astigmatic ratios, as applied to the target surface.

A further aspect of the invention provides a method as defined in claim 1.

Various methods and means are disclosed for varying the astigmatic ratio as applied to the surface. In one approach, the cylindrically-powered, optical system is movable along the optical path of the laser beam, thereby altering the astigmatic ratio. Alternatively, different lens or other optical elements (such as cylindrical mirrors) can be substituted for each other to vary the astigmatic ratio as applied to the corneal surface. In this aspect, the focal length of the cylindrically-powered, optical system is selected to alter one dimension of the irradiation ellipse delivered to the surface.

In yet another aspect, the invention provides a means for rotating the cylindrically-powered, optical system to orient the axes of the irradiation ellipse to coincide in an inverse manner with the patient's astigmatic axes.

According to a further aspect of the invention, in a laser system for eroding a surface astigmatically, there is provided a laser means for generating continuous or pulsed laser radiation of photoablative energy. This laser radiation is optically aligned for delivery to a target surface for photoablation. A beam control mechanism adjusts the spatial dimensions of the laser radiation through an iris diaphragm or the like, preferably driven by computer means. The laser energy is further aligned through a cylindrical optical means for adjusting the beam dimensions in one axis of the laser radiation, thereby forming an elliptical shape on the target surface.

In another aspect, the cylindrical optical means can be rotated to rotate the elliptical pattern of laser energy at the target surface for selecting and orienting the astigmatic erosion axis.

According to the invention, the cylindrical optical means can be axially shifted to change the size of the elliptical pattern of laser energy at the target surface to select an astigmatic erosion ratio on the surface.

The advantages presented by the features and aspects of the invention are several. In particular, the invention provides an elliptical irradiation pattern to the target surface, enabling simultaneous and bi-powered astigmatic erosion. The inclusion of a cylindrically-powered, optical system within the laser delivery system allows for easy polar orientation and the selection of the astigmatic ablation on a target surface. Applications, such as excimer laser keratomileusis, can take full advantage of the apparatus of the invention by ensuring proper axial orientation of the astigmatic correction applied to the myopic cornea: the accuracy of the orientation is equivalent to the accuracy in which the cylindrically-powered, optical system can be rotated. Furthermore, various astigmatic ratios can be achieved through the selection of the focal length of the cylindrically-powered, optical system; and variable and continuous astigmatic ratios can be achieved through the axial movement of the cylindrical system along the laser beam axis. The normal keratomileusis procedures can then be followed by adjusting the iris diameter of the apparatus of the invention to achieve the desired optical correction on the cornea. (The term "adjustable iris" as used herein is intended to encompass various systems for modifying the size of the laser beam, including, for example, adjustable diaphragms, aperture wheels, movable stops and other light restricting mechanisms, as well as optical elements associated therewith to maintain beam homogeneity.)

The invention will next be described in connection with certain preferred embodiments; however, it should be clear that various additions, subtractions and modifications can be made by those skilled in the art without departing from the scope of the invention. For example, the apparatus of the invention can be used in connection with congenital or other post-operative (e.g., cataract, or penetrating keratoplasty) astigmatisms.

## Brief Description of the Drawings

A more complete understanding of the invention may be obtained by reference to the drawings in which:

FIG. 1 is a diagrammatic illustration of apparatus for practicing a method of eroding a target surface astigmatically, in accordance with the invention;

FIG. 2 illustrates the elliptical laser beam irradiation used to erode a surface astigmatically;

FIGS. 3A through 3F illustrate diagrammatically the successive steps needed to etch a target surface astigmatically with elliptical irradiation;

FIGS. 4A and 4B illustrate curvatures created in orthogonal axes through surface erosion by elliptical laser irradiation;

FIGS. 5A and 5B are perspective views of an astigmatic myopic corneal surface, before and after reprofiling, respectively; and

FIGS. 6 and 7 give a more detailed schematic view of laser ablation systems in accordance with the invention.

Detailed Description

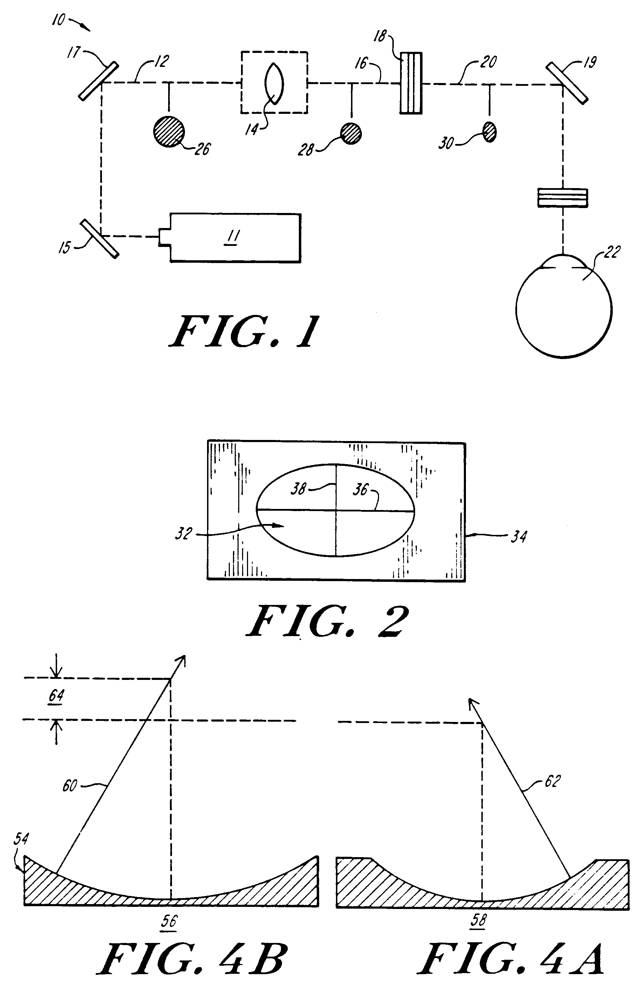

FIG. 1 illustrates a system 10 according to the invention for delivering an elliptical beam of ablative laser energy to a target surface. In FIG. 1, a laser means 11 provides radiation output 12 to a beam control optical system 14 which modifies the shape and size of the laser beam 16. This beam is aligned to a cylindrically-powered, optical system 18 to uniaxially modify the size and shape of the laser beam 20 for delivery to the target surface 22. FIG. 1 also shows cross-sectional views 24 of the laser beam as it is transformed into its various shapes 12, 16, 20. The beam 12, leaving the laser means 11, will typically have a circular beam shape 26. The beam 16, leaving the beam control optical system 14, will typically have a circular beam shape 28. The beam 20 leaving the cylindrically-powered, optical system 18 will form an elliptical pattern 30 on the target surface 22, as shown in its cross-sectional view 32.

The optical systems 14 and 18 are coaxially aligned to the laser means 11 so as to provide optimum and unaberrated throughput to the target surface 22. Mirrors 15, 17 and 19 permit the system to operate in a folded configuration. The laser means 11 can provide continuous or pulsed laser radiation output 12 to the beam control optical system 14. An afocal beam expander with a controllable aperture stop can comprise the beam control optical system 14 to change the cross-sectional diameter of the laser beam 12 into a larger or smaller size (e.g., the illustrated view 28 shows a smaller size) and to provide highly collimated output for the cylindrically-powered, optical system 18. This cylindrical system 18 provides optical power in one axis only, and modifies the laser beam 16 from a circular profile 28 to an elliptical one 30. Both systems 14 and 18 can have controllable aperture stops to adjust the beam dimensions delivered to the target surface 22. Each system controls one dimension. By adjustment of the two aperture stops, the major and minor axis dimension of the elliptical beam 30 can be adjusted, thereby fitting the astigmatic dimensions of the target surface 22.

The construction of the system shown in FIG. 1 can be made in several ways. Both optical systems 14 and 18 can be made of simple elements. For example, the beam control optical system can comprise a simple lens with an adjustable iris disposed to control the symmetrical beam shape 28. Alternatively, the beam control system 14 can also include a beam clipping mechanism, a light homogenizer and/or a beam expander, or these functions, if desired, can be performed by separate optical elements prior to beam shaping in optical system 14. The cylindrical system 18 can, likewise, be made of a simple lens, like a cylindrical lens for mirror, or can, likewise, include additional beam conditioning elements. This system 18 can be used to adjust one dimension of the elliptical laser profile 30 at the target surface 22.

Since the cylindrically-powered system 18 provides optical power in one axis only, one method of adjusting the elliptical dimension is to axially shift the system 18. The system 18 will cause the beam 16 to diverge, if the system 18 is negatively powered. Thus, shifting the system 18 axially will cause one dimension of the ellipse 30 to change on the target surface 22, since the beam 20 will diverge or converge over some distance to reach the surface 22. With accurate control of the axial movement, accurate sizing of the elliptical shape 30 can be achieved. The elliptical shape provides the means to etch a surface astigmatically.

The orientation of the ellipse 30 is also controllable, according to the methodology presented in accordance with the invention. As depicted in FIG. 1, the major axis of the ellipse 30 is horizontal. In the illustration, such a pattern can be created either through a negatively-powered, cylindrical system 18 in the horizontal axis or through a positively-powered, cylindrical system 18 in the vertical axis. In either case, when the system 18 is rotated about the optical axis of the laser beam 16 and 20, the orientation of the ellipse also changes. According to this technique, the ellipse can be oriented to fit the desired astigmatic axis on the target surface 22.

It should be appreciated that systems 14 and 18 can be combined in some implementations. The combined optical system would simply require that at least one optical element has astigmatic power. The combined system is axially shifted in the same manner and disposed with a single aperture stop to create the ellipse 30 in a controller manner. Alternatively, the combined system can be used in conjunction with a series of apertures, e.g., on a wheel or the like. Furthermore, rotating the combined system will also adjust the orientation of the ellipse on the target surface 22.

The overall system of FIG. 1 can be used in excimer laser keratomileusis. The laser means is a rapidly pulsed UV laser source, and the target surface is the human cornea, optically aligned to the laser means. The laser means, for example, can be an excimer laser, and one preferred embodiment is an Argon-Fluoride laser having a characteristic emission wavelength of about 193 nanometers. Other pulsed UV lasers having both shorter wavelengths down to about 157 nm (e.g., a Fluoride laser) and longer wavelengths up to about 300 nm.

Hereinafter, all methods for ablating a surface by laser energy described in relation to any method for treatment of a human or animal body by surgery or therapy are presented merely to illustrate the method claimed in claim 1, which excludes such methods, and to illustrate the uses to which the laser system of claims 3 to 8 may be put.

For example, in the case of eroding either Bowman's membrane or the stromal portion of the cornea by energy of wavelength 193 nm (the wavelength obtained from an ArF Excimer laser), the threshold value is about 50 mJ per $cm^2$ per pulse, and the saturation value is about 250 mJ per $cm^2$ per pulse. Suitable energy densities at the corneal surface are 50 mJ per $cm^2$ to one J per $cm^2$ per pulse for a wavelength of 193nm.

The threshold value will vary with wavelength, and at 157nm, which is the wavelength obtained from an $F_2$ laser, the threshold is about 5 mJ per $cm^2$ per pulse. At this wavelength, suitable energy densities at the corneal surface are 5 mJ per $cm^2$ to one J per $cm^2$ per pulse.

Most preferably, the laser system is used to provide an energy density at the surface to be eroded of slightly less than the saturation value. Thus, when eroding the cornea with a wavelength of 193nm (under which conditions the saturation value is 250mJ per $cm^2$ per pulse), it is preferable to provide to the cornea pulses of an energy density of 100 to 150 mJ per $cm^2$ per pulse. Typically, a single pulse will erode a depth in the range 0.1 to 1 micrometer of tissue from the cornea.

The pulse repetition rate for the laser may be chosen to meet the needs of each particular application. Normally the rate will be between 1 and 500 pulses per second, preferably between 1 and 100 pulses per second. When it is desired to vary the beam size, the laser pulses may be stopped. Alternatively, the beam size may be varied while the pulses continue. If a measurement device is used to monitor the erosion progress and control the laser system automatically, the beam size may be varied continuously at a controlled rate without interrupting the pulses.

Suitable irradiation intensities vary depending on the wavelength of the laser, and the nature of the irradiated object. For any given wavelength of laser energy applied to any given material, there will typically be a threshold value of energy density below which significant erosion does not occur. Above the threshold density, there will be a range of energy densities over which increasing energy densities give increasing depths of erosion, until a saturation value is reached. For increases in energy density above the saturation value, no significant increase in erosion occurs.

The threshold value and the saturation value vary from wavelength to wavelength of laser energy and from material to material of the surface to be eroded, in a manner which is not easily predictable. However, for any particular laser and any particular material, the values can be found readily by experiment.

With reference again to FIG. 1, the patient's astigmatic axis can be fit to the elliptical beam profile 30 through the rotation of the cylindrical system 18. An aperture stop disposed within the optical system 14 can control the one dimension of the ellipse 30 at the corneal target surface 22. Positioning the cylindrical system 18 axially can further control the other dimension of the ellipse 30. It is also preferable that the system 10 produce an output beam 20 having a substantially constant energy per unit area regardless of its varying size. For further details on basic laser keratomileusis systems and methods, as well as techniques for ensuring substantial constant energy density, see U.S. Patents 4,941,093 and 4,856,516.

The control of the dimension of the ellipse along the axis with the cylindrical power can also be achieved through the selection and/or replacement of particular optical elements with system 18. According to this embodiment, by selecting cylindrical elements with different optical powers (i.e., different focal lengths) for placement within the system shown in FIG. 1, variable elliptical dimensions can be achieved. This methodology is equivalent to adjusting the axial placement of the system 18.

FIG. 2 illustrates the elliptical shape 32 of the laser energy striking the target surface 34. The difference between major axis 36 and the minor axis 38 of the ellipse 32 provides the means to erode a surface astigmatically. To create the desired surface etch, the surface 34 is irradiated with a laser which has ablative properties while the dimensions of the ellipse are altered. The ratio between the major and minor axes is held constant while the overall area of the ellipse 32 is changed.

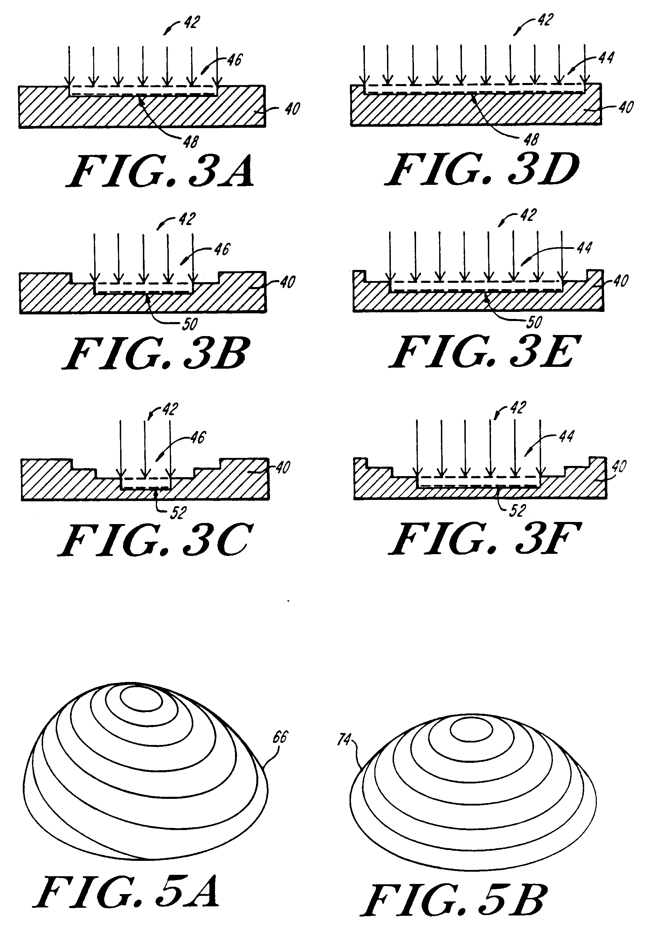

FIGS. 3A-3F illustrate how the elliptical pattern can erode a surface astigmatically. The target surface 40 is irradiated by the elliptical laser beam 42. The major axis 44 of the beam irradiates a wider dimension on the target surface 40 than does the minor axis 46. After a predetermined period of time, the laser beam etches away the part 48 of the target surface 40 which was under irradiation. Then the area of the ellipse is modified by reducing the size of the major axis 44 and minor axis 46, but at the same time keeping the ratio between the two constant. Typically, the laser beam 42 is inactivated or blocked by a shutter while the exposive area is adjusted. After another period of time, another part 50 of the target surface is removed through laser etching. The area of the ellipse is adjusted again, as described in FIG. 1, to etch another part 52 from the target surface 40. FIGS. 3A-3C illustrate the cumulative effect along one axis of the elliptical beam while FIGS. 3D-3F illustrate the cumulative effect along the other axis.

FIGS. 4A and 4B illustrate the end result of the process described in FIGS. 3A-3F, with successive elliptical irradiations on a planar target surface 54. The target surface has a larger diameter 56 etched pattern along the axis irradiated by the major axis of the ellipse and a smaller diameter 58 etched pattern on the minor axis of the ellipse. By reducing the area of the ellipse in a controlled manner and while keeping the ratio between the major and minor axis fixed, two distinct optical powers can be created on the target surface 54. As shown in FIG. 4A, the major axis has a larger radius of curvature 60, and as shown in FIG. 4B, the minor axis has a smaller radius of curvature 62. The difference between the two radii 64 is approximately a linear function of the amount of astigmatic power created on the surface 54 through the laser erosion.

FIGS. 5A and 5B illustrate how the cornea of an astigmatic, myopic eye can be reprofiled to achieve a desired optical power and spherical refraction. Consider the astigmatic cornea 66 shown in FIG. 5A as composed of a series of layers. Each layer can be removed with one or more laser pulses, depending upon the laser and material. After a layer is removed, the irradiation area is altered to remove a different sized layer in a successive irradiation. Controlling the size of the laser irradiation on the cornea through sequential layer etches creates the desired profile 74, shown in FIG. 5B. The contour lines, shown in both FIG. 5A and 5B, represent lines of equal topographic height on the corneal surface before and after treatment, respectively.

The varying aperture profile of successive laser irradiations is geometric. For one axis,

$$t = R - \sqrt{R^2 - (D^2/4)} \qquad (1)$$

where t is the height of the eye surface (measured from a base plane which intersects the eye at a diameter D), D is the outer diameter of the ablated surface area and R is the resultant, one-dimensional radius of the surface 66. The only portion of the surface 66 which is of interest is the dimension D, which is initially under the laser irradiation. Once t is determined, the successive sizes of the ellipse to remove each layer can be determined. For example, one laser pulse can etch away $\Delta t$ in thickness from the target surface 66. By replacing t with t - $\Delta t$, a smaller linear dimension D is determined.

The rate of change in the size of the elliptical exposure area depends upon the selected radius of curvature 76 for the surface erosion. For one dimension, the power of a surface can be described in terms of Diopters (1/meter units), the typical terminology for vision correction. A Diopter is defined as

$$\text{Diopter} = \frac{n'-n}{R} \qquad (2)$$

where n and n' are the wavelength dependent optical indexes of refraction before and within target surface 66, respectively. R is the radius of the surface. In excimer laser keratoplasty or keratomileusis procedures, the index n of the incident medium is air, with an index of approximately 1. The index of the average human cornea, i.e., n', is approximately 1.376 for visible light. If a patient needs a -3.0 Diopter correction, the radius of the surface erosion is about 125.33 mm. The successive irradiations on the cornea are, therefore, adjusted in size to erode the surface 66 to that radius.

If a patient has myopic astigmatism, or more commonly called astigmatic near-sightedness, both axes of the cornea can be eroded simultaneously to perform a bi-powered erosion. If, for example, a -3.0 Diopter correction is needed in one axis and a -3.9 Diopter correction is needed in the orthogonal axis on the cornea, the corresponding irradiation size of the elliptical excimer laser beam is about 4.6 mm and about 4.1 mm, respectively. The thickness t of the cornea removed during the ablation will vary depending upon size of the optical zone being reprofiled. The procedure can be largely confined to erosion of the Bowman's layer of the cornea, if desired, by choosing a small optical zone for reprofiling. Alternatively, a larger optical zone may be desired and, in such case, penetration into the stromal region of the cornea will typically occur. Generally, it is desirable to avoid ablation of more than 100-200 microns of the cornea, in any event.

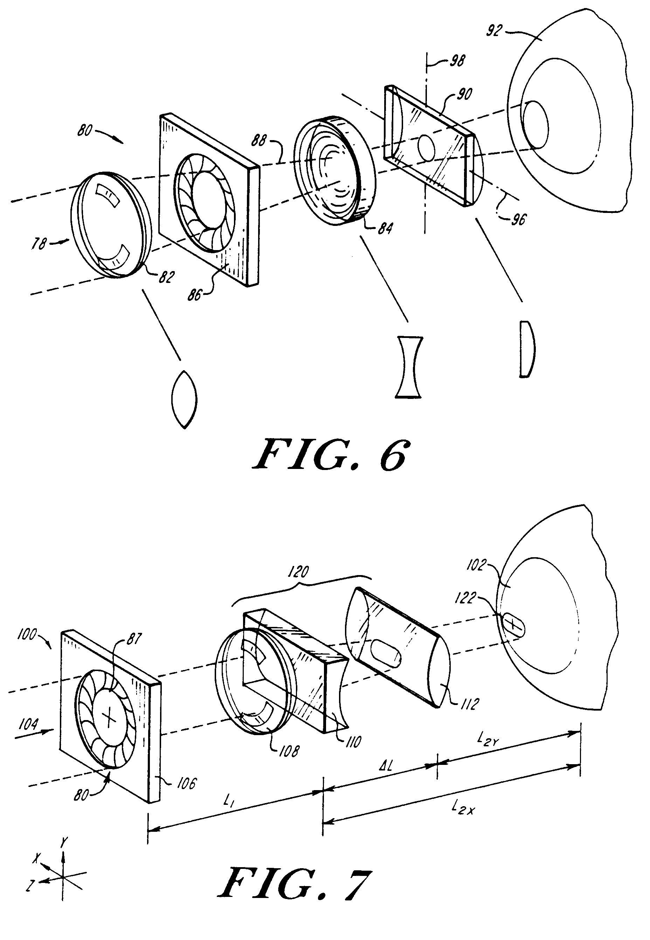

FIG. 6 is a more detailed illustration of one embodiment of the invention. A laser beam 78 enters a beam control optical system 80, which is optically aligned to the beam 78. The beam control optical system 80 is an afocal system, producing collimated radiation of a different radius than the input while maintaining substantially constant energy density regardless of the beam dimensions. A positive lens 82 and negative lens 84 positioned at a distance equal to the sum of their respective focal lengths can create such a system. An aperture 86 limits the diametrical extent 88 of the laser beam 78 transmitted through the beam control optical system 80. The laser beam 78 entering the cylindrically-powered, optical system 90 is collimated and circular where it is modified in one axis for delivery to the target surface 92. The aperture stop 86 can change the diameter of the laser beam 78 in several ways. For example, through a computer driven mechanism, the diameter of the aperture can be altered, e.g., by an adjustable iris diaphragm or the like. Or equivalently, the axial movement of the aperture 86 can alter the beam 78 diameter exiting the beam control optical system 80.

FIG. 6 also shows a construction of the cylindrically-powered, optical system 90. One axis 96 does not alter the size or shape of the laser beam 78. The other axis 98 contains a cylindrical correction power element. Varying astigmatic ratios can be achieved by adjusting the position of element 90 axially.

The polar orientation of the ellipse on the eye can be adjusted through rotation of the cylindrically-powered, optical system 90 about the laser beam 78 axis to fit the astigmatic axes of the surface or, for example, to fit the astigmatic axes of the myopic patient in keratoplasty or keratomileusis surgery.

FIG. 7 is another embodiment of the invention in which the system 100 not only delivers an elliptical beam of ablative laser radiation to a target surface 102 but also ensures that the image plane is unchanged for both optical axes (as defined by the orientation of cylindrical lenses 110 and 112). A laser beam 104 enters a beam control optical system 80 (which is simply illustrated as an adjustable iris 86 having an aperture 87, although it may include additional elements, such as matched positive and negative lenses as illustrated in FIG. 6, or a combination of lenses and an adjustable stop), again producing a collimated radiation output of a different radius than the input while maintaining substantially constant energy density regardless of the beam dimensions.

In FIG. 7, a cylindrically-power optical system 120 is employed to image the aperture of the beam control system 80 in a plane substantially parallel to the aperture plane (e.g., perpendicular to the beam axis). As illustrated the cylindrically powered optical system 120 includes a spherical lens 108, a cylindrical lens with negative focal power 110 and a complementary cylindrical lens with positive focal power 112. (Lenses 108 and 110 can, of course, be designed as a single lens with one surface spherical and the other cylindrical. This simplification would reduce the number of components and result in smaller losses of beam energy. However, for purposes of illustration and explanation of underlying principles, the lenses 108 and 110 are shown as two separate elements.)

For purposes of simplified explanation, assume that lenses 108 and 110 are either formed as a single unit or separated by negligible distance. The magnification, $M_x$, on the X axis, which is not affected by cylindrical lenses 110 and 112, is defined by the following formula

$$M_x = \frac{L_{2X}}{L_1} \qquad (3)$$

In one preferred embodiment, spherical lens 108 and cylindrical lens 110 are chosen, such that their combined focal power on the Y axis is equal to $L_1$. If lenses 108 and 110 are close to each other, the focal length of cylindrical lens 110 (which will be negative) can be defined by the following equation.

$$f_{110} = \frac{1}{1/L_1 - 1/f_{108}} \qquad (4)$$

This results in "parallel beam processing" along the Y axis in the region between the first (negative) cylindrical lens 110 and the second (positive) cylindrical lens 112. The distance ΔL between lens 110 and lens 112 is then chosen, such that the length of the final span $L_{2Y}$ between lens 112 and the target is chosen.

The distance ΔL between lenses 110 and 112 is chosen, such the remaining distance $L_{2Y}$ between lens 112 and the target is defined as follows:

$$f_{112} = L_{2y} \qquad (5)$$

and the magnification along the Y axis $M_x$ is:

$$M_x = \frac{L_{2y}}{L_1} \qquad (6)$$

which will always be less than $M_x$.

It should be clear that the use of spherical lens 108, and the two cylindrical lenses 110 and 112, permits the clinician to rotate the axis of cylindrically around Z axis while maintaining the image of aperture in a plane substantially parallel to the plane of aperture 87. In general, the use of two cylindrical lenses of opposite power will allow this afocal magnification effect so long as both cylindrical lenses are sufficiently separated from each other.

The image spot 122 shows schematically that the dimension of the image in the Y axis is smaller than the dimension in the X axis. By rotating the entire assembly 120, the asymmetry can be fitted to any particular astigmatic axis, to selectively ablate the cornea and correct for a particular astigmatic problem.

It should also be clear that changes in the image magnification $M_y$ can be accomplished by either axial movement of one or more lens elements, or by swapping one cylindrical lens, (e.g., lens 112), with another having a different focal power and positioning the lens to satisfy the conditions outlined above. In essence, the free space between lenses 110 and 112 is a buffer in which to accommodate variations in the Y axis magnification.

Since swapping lens element 112 for another discrete lens element may be difficult or time consuming in practice, continuous variation of My also can be accomplished by replacing the simple lens 112 with a variable cylindrical zoom lens.

## Claims

1. A method for ablating a surface astigmatically by laser energy, the method comprising the steps of:

   aligning a surface (22,92,102) with a laser means (11) e.g. an excimer laser, which is operable to deliver a beam (12) of photoablative pulses of laser energy along a path to the surface (22,92,102);
   varying the size of the beam (12);
   disposing a cylindrical optical element (90,120), e.g. a cylindrical lens, along said beam path;

the cylindrical optical element applying a cylindrical power to the beam (16) to form a time-varying, elliptical distribution of energy on said surface (22,92,102); and shifting the axial position of said cylindrical optical element (90,120) along said beam path to select an astigmatic ratio on said surface (22,92,102) for the astigmatic ablation;

subject to a disclaimer of methods for treatment of a human or animal body by surgery or therapy.

2. The method of claim 1 wherein the method further comprises rotating the cylindrically-powered, optical element (90,120) to select the orientation of the orthogonal axes on said surface (22,92,102) for the astigmatic erosion.

3. A laser system for astigmatically reprofiling a surface, said laser system comprising:

laser means (11) for generating pulses of laser light (12) along a beam path at an energy level, such that the pulses can be absorbed at a surface (22,92,102) to induce photoablation;
beam control system, (14,80,100) for controlling said beam dimensions at said surface (22,92,102); and
cylindrically-powered, optical means (18) comprising at least one cylindrically-powered element (90,120) for adjusting said beam dimensions in one axis for creating an irradiation pattern at said surface (22); and
in which said irradiation pattern is an astigmatic irradiation pattern;
characterized in that:
said cylindrically-powered, optical means (90,120) further comprises means for shifting along said beam path said it least one cylindrically powered element (90,120) to select an astigmatic ratio to be applied to said surface (22,92,102).

4. A laser system according to claim 3 wherein said beam control system (14,80,100) includes an adjustable iris (86) for controlling the diameter of said laser beam (12).

5. A laser system according to claim 3, wherein said cylindrically-powered, optical means (18) further comprises means for rotating the said at least one cylindrically-powered, optical element (90,120) to orient an astigmatic axis on said surface (92,102).

6. A laser system according to claim 3 wherein said cylindrically-powered, optical means (120) images an aperture (87) of said beam control system (80) in a plane substantially parallel to the plane of the aperture.

7. A laser system according to claim 3 wherein said beam control system (14) is afocal and further includes a beam stop which is movable along said laser beam axis for controlling the diameter of said laser beam (12) applied to said surface (22,92,102).

8. A laser system according to claim 3 wherein the laser means (11) is an excimer laser, for example an Argon Fluoride laser.

**Patentansprüche**

1. Verfahren zur astigmatischen Ablation einer Oberfläche mittels Laserenergie, wobei das Verfahren die Schritte aufweist:

Ausrichten einer Oberfläche (22, 92, 102) zu einer Lasereinrichtung (11), z.B. einem Excimer-Laser, welche so betreibbar ist, daß sie einen Strahl (12) photoablativer Laserenergieimpulse entlang eines Pfades zu der Oberfläche (22, 92, 102) liefert;
Verändern der Größe des Strahls (12);
Anordnen eines zylindrischen optischen Elementes (90, 120), z.B. einer zylindrischen Linse, entlang dieses Strahlpfades;
wobei das zylindrische optische Element eine zylindrische Brechwirkung auf den Strahl (16) so ausübt, daß sie eine zeitlich-variierende elliptische Energieverteilung auf der Oberfläche (22, 92, 102) erzeugt; und
verschieben der axialen Position des zylindrischen optischen Elementes (90, 120) entlang des Strahlpfades, um ein astigmatisches Verhältnis auf der Oberfläche (22, 92, 102) für die astigmatische Ablation zu wählen;
unter Erklärung des Verzichts auf Verfahren zur Behandlung eines menschlichen oder tierischen Körpers mittels Chirurgie oder Therapie.

2. Verfahren nach Anspruch 1, wobei das Verfahren weiterhin ein Drehen des mit zylindrischer Brechwirkung versehenen optischen Elementes (90, 120) umfaßt, um die Orientierung der orthogonalen Achsen auf der Oberfläche (22, 92, 102) für die astigmatische Erosion zu wählen.

3. Lasersystem zur astigmatischen Neuprofilierung einer Oberfläche, wobei das Lasersystem aufweist:

eine Lasereinrichtung (11) zum Erzeugen von Laserlichtimpulsen (12) entlang eines Strahlpfades bei einem Energiepegel in der Weise, daß die Impulse auf einer Oberfläche (22, 92, 102) absorbiert werden können, um eine Photoablation zu induzieren;

ein Strahlsteuersystem (14, 80, 100) zum Steuern der Strahlabmessungen auf der Oberfläche (22, 92, 102); und

eine mit zylindrischer Brechwirkung versehene optische Einrichtung (18), welche wenigstens ein mit zylindrischer Brechwirkung versehenes optisches Element (90, 120) für die Anpassung dieser Strahlabmessungen in einer Achse zum Erzeugen eines Bestrahlungsmusters auf der Oberfläche (22) aufweist, und in welcher dieses Bestrahlungsmuster ein astigmatisches Bestrahlungsmuster ist;

dadurch gekennzeichnet, daß

die mit zylindrischer Brechwirkung versehene optische Einrichtung (18) ferner eine Einrichtung zum Verschieben des wenigstens einen mit zylindrischer Brechwirkung versehene optischen Elementes (90, 120) entlang des Strahlpfades aufweist, um ein an die Oberfläche (22, 92, 102) anzulegendes astigmatisches Verhältnis zu wählen.

4. Lasersystem nach Anspruch 3, wobei das Lasersteuersystem (14, 80, 100) eine einstellbare Blende (86) zur Steuerung des Durchmessers des Laserstrahls (12) enthält.

5. Lasersystem nach Anspruch 3, wobei die mit zylindrischer Brechwirkung versehene optische Einrichtung (18) weiterhin eine Einrichtung zum Drehen des wenigsten einen mit zylindrischer Brechwirkung versehenen optischen Elementes (90, 120) zur Ausrichtung einer astigmatischen Achse auf der Oberfläche (92, 102) aufweist.

6. Lasersystem nach Anspruch 3, wobei die mit zylindrischer Brechwirkung versehene optische Einrichtung (120) eine Apertur (87) des Strahlsteuersystems (80) in einer Ebene abbildet, die im wesentlichen zu der Ebene der Apertur parallel liegt.

7. Lasersystem nach Anspruch 3, wobei das Strahlsteuersystem (14) afokal ist und weiterhin einen Strahlbegrenzer enthält, welcher zur Steuerung des Durchmessers des auf die Oberfläche (22, 92, 102) aufgebrachten Laserstrahls (12) entlang der Laserstrahlachse bewegbar ist.

8. Lasersystem nach Anspruch 3, wobei die Lasereinrichtung (11) ein Excimer-Laser, beispielsweise ein Argonfluorid-Laser ist.

**Revendications**

1. Procédé pour réaliser une ablation d'une surface de manière astigmatique par une énergie laser, le procédé comprenant les étapes consistant à :

aligner la surface (22, 92, 102) avec des moyens laser (11), par exemple un laser excimer, qui peut fonctionner pour délivrer un faisceau (12) d'impulsions de photo-ablation d'énergie laser le long d'un trajet vers la surface (22, 92, 102) ;

faire varier la taille du faisceau (12) ;

disposer un élément optique cylindrique (90, 120), par exemple une lentille cylindrique, le long dudit trajet de faisceau ;

l'élément optique cylindrique appliquant une puissance cylindrique au faisceau (16) pour former une distribution d'énergie elliptique et variant dans le temps sur ladite surface (22, 92, 102); et

décaler la position axiale dudit élément optique cylindrique (90, 120) le long dudit trajet du faisceau pour sélectionner un rapport astigmatique sur ladite surface (22, 92, 102) pour une ablation astigmatique ;

à l'exclusion des méthodes de traitement pour le corps humain ou animal par une intervention chirurgicale ou thérapeutique.

2. Procédé selon la revendication 1, dans lequel le procédé comprend en outre l'étape consistant à faire tourner l'élément optique à puissance cylindrique pour sélectionner l'orientation des axes orthogonaux sur ladite surface (22, 92, 102) pour une érosion astigmatique.

3. Système laser pour reprofiler de manière astigmatique une surface, ledit système laser comprenant :

des moyens laser (11) pour générer des impulsions de lumière laser (12) le long d'un trajet de faisceau à un niveau d'énergie tel que les impulsions peuvent être absorbées sur la surface (22, 92, 102) pour induire une photo-ablation ;

un système de commande de faisceau (14, 80, 100) pour commander lesdites dimensions de faisceau au niveau de ladite surface (22, 92, 102) ; et

des moyens optiques à puissance cylindrique comprenant au moins un élément à puissance cylindrique (90, 120) pour ajuster lesdites dimensions de faisceau selon un axe pour créer un motif d'irradiation sur ladite surface (22) ; et

dans lequel ledit motif d'irradiation est un motif d'irradiation astigmatique,

caractérisé en ce que lesdits moyens optiques à puissance cylindrique (90, 120) comprennent en outre des moyens pour décaler le long dudit trajet de faisceau ledit au moins un élément (90, 120) à puissance cylindrique pour sélectionner un rapport astigmatique à appliquer à ladite surface (22, 92, 102).

4. Système laser selon la revendication 3, dans lequel le système de commande du faisceau (14, 80, 100) comprend un diaphragme à iris (86) ajustable pour commander le diamètre dudit faisceau laser (12).

5. Système laser selon la revendication 3, dans lequel les moyens (18) optiques à puissance cylindrique comprennent en outre des moyens pour faire tourner ledit au moins un élément optique à puissance cylindrique (90, 120) pour orienter un axe astigmatique sur ladite surface (92, 102).

6. Système laser selon la revendication 3, dans lequel les moyens (120) optiques à puissance cylindrique projettent une image d'une ouverture de diaphragme (87) du système (80) de commande du faisceau dans un plan sensiblement parallèle au plan de l'ouverture de diaphragme.

7. Système laser selon la revendication 3, dans lequel le système de commande du faisceau (14) est afocal et comprend en outre un dispositif d'arrêt de faisceau qui est déplaçable le long dudit axe du faisceau laser pour commander le diamètre dudit faisceau-laser (12) appliqué à ladite surface (22, 92, 102).

8. Système laser selon la revendication 3, dans lequel les moyens laser (11) sont un laser excimer, par exemple un laser à argon fluoride.

FIG. 1

FIG. 2

FIG. 4B          FIG. 4A

FIG. 3A

FIG. 3D

FIG. 3B

FIG. 3E

FIG. 3C

FIG. 3F

FIG. 5A

FIG. 5B

## FIG. 6

## FIG. 7